(19) Europäisches Patentamt
European Patent Office
Office européen des brevets

(11) Publication number: 0 107 907 A2

# (12) EUROPEAN PATENT APPLICATION

(21) Application number: 83305612.0

(22) Date of filing: 22.09.83

(51) Int. Cl.[3]: C 12 P 21/02
C 07 C 103/52, C 07 F 9/38
A 61 K 37/16
//(C12P21/02, C12R1/465)

(30) Priority: 27.09.82 US 424805
17.02.83 US 467419

(43) Date of publication of application:
09.05.84 Bulletin 84/19

(84) Designated Contracting States:
AT BE CH DE FR GB IT LI LU NL SE

(71) Applicant: ELI LILLY AND COMPANY
307, East McCarty Street
Indianapolis Indiana 46285(US)

(72) Inventor: Johnson, Ronald Doyle
431, Blue Ridge Road
Indianapolis Indiana 46208(US)

(72) Inventor: Gordee, Robert Stouffer
5166 East 74th Place
Indianapolis Indiana 46250(US)

(72) Inventor: Kastner, Ralph Emil
243 Webb Drive
Indianapolis Indiana 46227(US)

(72) Inventor: Larsen, Stephen Hans
6459, Bayside South Drive
Indianapolis Indiana 46250(US)

(72) Inventor: Ose, Earl Eugene
805 Oak Court, R.R.7
Greenfield Indiana 46140(US)

(74) Representative: Crowther, Terence Roger et al,
Erl Wood Manor
Windlesham Surrey GU20 6PH(GB)

## (54) Improvements in or relating to immunomodulating agents.

(57) Compounds having the formula:

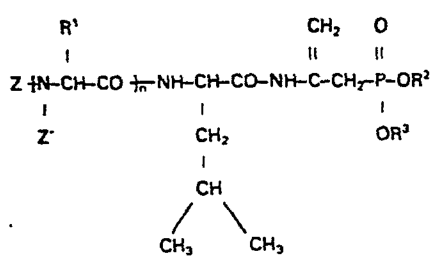

2

wherein

$R^1$ represents the characterizing group of an α-amino acid of the type normally found in proteins;

$R^2$ and $R^3$ are, independently, hydrogen or $C_1$-$C_3$-alkyl;

Z is hydrogen, $C_1$-$C_6$-alkyl, $C_1$-$C_6$-alkanoyl, phenyl-$C_1$-$C_3$-alkyl, phenyl-$(X)_m$-$C_1$-$C_3$-alkanoyl, or an amino-protecting group.

Z' is hydrogen or $C_1$-$C_6$-alkyl;

X is oxygen or sulfur;

m is 0 or 1; and

n is 0, 1, 2 or 3;

provided that when $R^2$ and $R^3$ are both alkyl, they must be identical; and the salts thereof, are disclosed herein and are useful as immunoregulating agents. The compounds of formula *2* are prepared from antibiotic A53868 factor A by conventional peptide synthesis. Also disclosed as immunoregulating agents are fosfomycin and its derivatives.

EP 0 107 907 A2

# IMPROVEMENTS IN OR RELATING TO IMMUNOMODULATING AGENTS

This invention relates to new antibiotics and immunomodulating agents. This invention also relates to the discovery that certain known antibiotics can be used as immunomodulators.

New, improved antibiotics are continually in demand. Better antibiotics are needed for treating human diseases, and improved antibiotics are also needed in the veterinary field. Increased potency, expanded spectrum of bacterial inhibition, increased *in vivo* efficacy, and improved pharmaceutical properties (such as greater oral absorption, higher blood or tissue concentrations, longer body half life, and more advantageous rate or route of excretion and rate or pattern of metabolism) are some of the goals for improved antibiotics.

The body's defense system (the immune system) is exceedingly complex, and understanding its mechanisms is an important goal. Although knowledge in this area has expanded at a rapid rate in the past decade, there is much to learn. Macrophages play an important role in the immune system. These cells are both phagocytic (they can engulf and kill microbial cells) and secretory (they release chemical signals which diffuse throughout the body). Macrophages are able to carry (or at least are able to make) more than 50 destructive or instructive secretory products, and are sometimes able to destroy tumor cells completely. Thus, immunomodulating agents which act by activating macrophage cells are quite valuable.

The state of the art for immunomodulators is shown by H. Umegawa, "Recent Studies on Antibiotics and Small Molecular Immunomodulators with Potential Usefulness in Treating Lung Cancer:Part II--Small Molecular Weight Immunomodulators Produced by Microorganisms," Inter. J. Clin. Pharmacol. Ther. and Toxicol. 20 (1), 19-23 (1982).

U.S. Patent No. 4,331,591 discloses a chemical process for the preparation of peptide derivatives of certain α-aminophosphonic acids. The derivatives potentiate the activity of antibiotics such as penicillin, cephalosporins, and D-cycloserine. The reference compounds are structurally and functionally different from the present compounds.

This invention provides compounds which are designated antibiotic A53868 factor A, which is $N^2$-glycyl-N-(1-methylene-2-phosphonoethyl)leucinamide having formula 1:

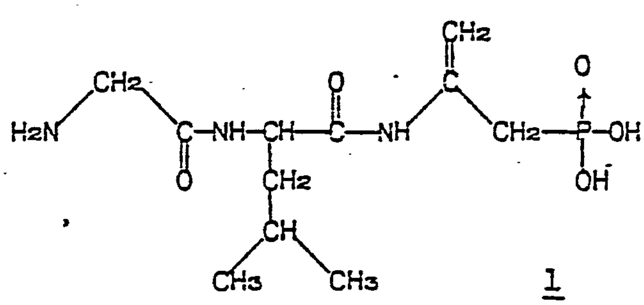

1

Antibiotic A53868 factor A is a new member of a group of phosphonopeptide antibiotics. Members of this group include alafosfalin (L-alanyl-L-1-aminoethylphosphonic acid) [see Antimicrobial Agents and Chemotherapy 18 (6), 897-905 (1980)], FR-31564 [see

ibid 19 (6), 1013-1023 (1981)], and others [see European Patents 26-409 and 26-410 and Japanese Patents J5 6051-494 and J5 6156-294].

The compounds of this invention have formula 2:

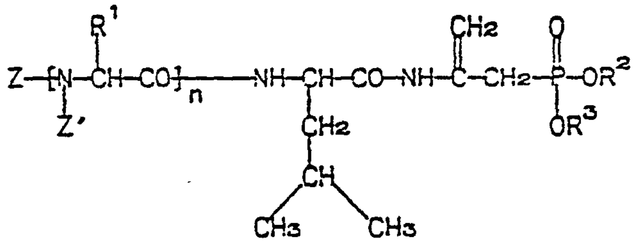

2

wherein

$R^1$ represents the characterizing group of an α-amino acid of the type normally found in proteins;

$R^2$ and $R^3$ are, independently, hydrogen or $C_1$-$C_3$-alkyl;

Z is hydrogen, $C_1$-$C_6$-alkyl, $C_1$-$C_6$-alkanoyl, phenyl-$C_1$-$C_3$-alkyl, phenyl-$(X)_m$-$C_1$-$C_3$-alkanoyl, or an amino-protecting group;

Z' is hydrogen or $C_1$-$C_6$-alkyl;

X is oxygen or sulfur;

m is 0 or 1; and

n is 0, 1, 2 or 3;

provided that when $R^2$ and $R^3$ are both alkyl, they must be identical; and the salts thereof.

The compound of formula 2 where n is 1 and $R^1$, $R^2$, $R^3$, Z and Z' are hydrogen is the compound of formula 1 above.

A preferred group of compounds of formula 2 is when Z' is alkyl, and Z must also be alkyl.

A further preferred group of compounds of formula 2 is the derivatives of the compounds of formula 1, having the formula 2a:

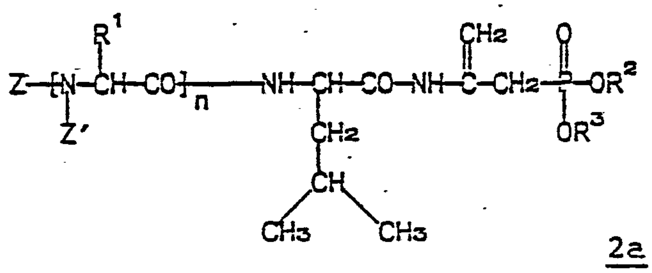

2a

wherein

$R^1$ represents the characterizing group of an α-amino acid of the type normally found in proteins;

$R^2$ and $R^3$ are, independently, hydrogen or $C_1$-$C_3$-alkyl;

Z is hydrogen, $C_1$-$C_6$-alkyl, $C_1$-$C_6$-alkanoyl, phenyl-$C_1$-$C_3$-alkyl, phenyl-$(X)_m$-$C_1$-$C_3$-alkanoyl, or an amino-protecting group;

Z' is hydrogen or $C_1$-$C_6$-alkyl;

X is oxygen or sulfur;

m is 0 or 1; and

n is 0, 1, 2 or 3;

provided that: 1) when $R^2$ and $R^3$ are both alkyl, they must be identical; and 2) when n is 1, one of $R^1$, $R^2$, $R^3$, Z or Z' must be other than hydrogen; and the salts thereof.

The compounds of formula 2 are useful also as antibiotics and/or as intermediates to antibiotics. Methods of treating certain infections with, and pharmaceutical compositions comprising, a compound of formula 2 or a pharmaceutically-acceptable salt thereof are also provided by this invention.

This invention further provides methods of regulating the immune system by activating macrophage cells with a compound of formula 1, 2, or 3:

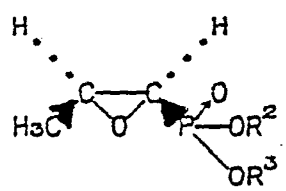 ,

3

wherein $R^2$ and $R^3$ are as defined above, and the salts thereof.

The process for preparing A53863 factor A comprises cultivating *Streptomyces luridus* NRRL 15101 or a mutant or recombinant thereof which produces A53868 factor A in a culture medium containing assimilable sources of carbon, nitrogen, and inorganic salts, under submerged aerobic fermentation conditions until a substantial amount of antibiotic activity is produced.

A53863 factor A can be isolated from the reaction mixture by conventional techniques. The product of formula 1 can be further reacted by conventional techniques to provide the compounds of formula 2a above. These techniques include:

derivatizing a compound of formula 2a wherein at least one of $R^2$, $R^3$, Z or Z' is not hydrogen;

reacting a compound of formula 1 by solution phase peptide synthesis to provide compounds of formula 2a wherein $R^1$ is hydrogen and Z or Z' is not hydrogen; and

reacting a compound of formula 1 with leucine amino peptidase or selective acid hydrolysis, followed by conventional solution phase peptide synthesis to provide the compounds of formula 2a above.

In this specification, the following abbreviations, most of which are well known, are used:

Ala - alanine
Arg - arginine
Asp - aspartic acid
Asx - asparagine or aspartic acid
Cys - cysteine
Gln - glutamine
Glu - glutamic acid
Glx - glutamine or glutamic acid
Gly - glycine
Hse - homoserine
Ile - isoleucine
Leu - leucine
Lys - lysine
Met - methionine
Nle - norleucine
Nva - norvaline
Pro - proline
Phe - phenylalanine
Ser - serine
Thr - threonine

Trp - tryptophan
Tyr - tyrosine
Val - valine

The abbreviation LAPP (for leucylaminopropenylphosphonate) will be used for the moiety 4, which is common to the compounds of formulae 1 and 2 and which has the formula:

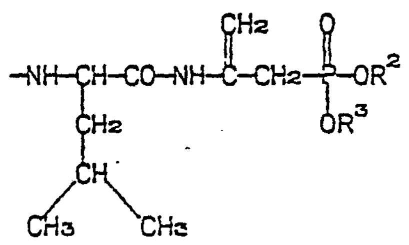

4

wherein
$R^2$ and $R^3$ are, independently, hydrogen or $C_1$-$C_3$-alkyl;
provided that: when $R^2$ and $R^3$ are both alkyl, they must be identical.

The compounds of this invention can be shown by formula 2b which is:

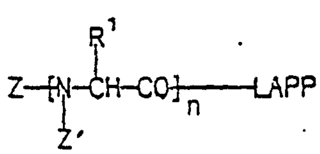

2b

wherein
LAPP is formula 4 above;

$R^1$ represents the characterizing group of an α-amino acid of the type normally found in proteins;

Z is hydrogen, $C_1$-$C_6$-alkyl, $C_1$-$C_6$-alkanoyl, phenyl-$C_1$-$C_3$-alkyl, phenyl-$(X)_m$-$C_1$-$C_3$-alkanoyl, or an amino-protecting group;

Z' is hydrogen or $C_1$-$C_6$-alkyl;

X is oxygen or sulfur;

m is 0 or 1; and

n is 0, 1, 2 or 3;

and the salts thereof.

The term "the characterizing group of an α-amino acid of the type normally found in proteins" refers to the residue $R^1$ in a natural alpha-amino acid of the general formula

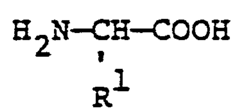

which is of the type normally occurring in proteins. Examples of such amino acids and the corresponding $R^1$ groups are: alanine ($R^1$ = methyl); leucine ($R^1$ = isobutyl); glutamic acid ($R^1$ = 2-carboxyethyl). In addition, $R^1$ can represent a residue linked to the amino nitrogen (with the concomitant loss of one of the hydrogen atoms attached to the nitrogen), thus forming a nitrogen-containing ring such as occurs in proline.

The terms "$C_1$-$C_6$-alkyl" and "$C_1$-$C_3$-alkyl" as used herein mean a straight- or branched-chain alkyl group containing from one to six or one to three carbon atoms, respectively. Such groups include methyl,

ethyl, isopropyl, *n*-butyl, *sec*-butyl, isobutyl, *tert*-butyl, *n*-pentyl, isopentyl, and the like.

The term "$C_1$-$C_6$-alkanoyl" as used herein means an acyl moiety derived from a carboxylic acid containing from one to six carbon atoms. In such a moiety, the alkyl group can be straight, branched, or cyclic. Acetyl, propionyl, *n*-butyryl, isobutyryl, *n*-valeryl, and isovaleryl are examples of such groups.

The terms "phenyl-$C_1$-$C_3$-alkyl" and "phenyl-$(X)_m$-$C_1$-$C_3$-alkanoyl" refer to groups such as benzyl, phenethyl, benzoyl, phenylacetyl, phenylpropionyl, phenoxyacetyl and phenylthioacetyl.

The term "amino-protecting group" is known to those in the art. Examples of suitable protecting groups can be found in "Protective Groups in Organic Synthesis" by Theodora W. Greene, John Wiley and Sons, New York, 1981, Chapter 7.

Antibiotic A53868 factor A is a member of an antibiotic complex which comprises more than one factor. The A53868 complex contains major factor A and other as yet uncharacterized factors.

The term "complex" as used in the fermentation art and in this specification refers to a mixture of co-produced individual antibiotic factors. As will be recognized by those familar with antibiotic production by fermentation, the number and ratio of individual factors produced in an antibiotic complex will vary, depending upon the fermentation conditions used. In the A53868 complex, factor A is the major factor.

The following paragraphs describe the properties of A53868 factor A.

A53868 factor A is a colorless amorphous material having an empirical formula of $C_{11}H_{22}N_3O_5P$ and a molecular weight of about 307. Elemental analysis of A53868 factor A indicates that it has the following approximate percentage composition:

| | Found | | Calculated for $C_{11}H_{21}N_3O_5PNa$ |
|---|---|---|---|
| | Sample I | Sample II | |
| Carbon | 41.32 | 38.74 | 40.12 |
| Hydrogen | 7.61 | 6.78 | 6.43 |
| Nitrogen | 11.53 | 12.42 | 12.76 |
| Oxygen | 24.38 | -- | 24.30 |
| Phosphorus | -- | 7.70 | 9.41 |
| Ash* | 13.58 | -- | -- |

*Ash was shown to be phosphate

Mass spectrometry of A53868 factor A, run in the fast-atom bombardment mode, gave the following results:

| | m/Z | HRMS | Elemental Composition |
|---|---|---|---|
| M+H | 308 | 308.13818 | $C_{11}H_{23}N_3O_5P$ |
| | 143 | 143.11878 | $C_7H_{15}N_2O$ |
| | 138 | 138.03240 | $C_3H_9NO_3P$ |

Molecular weight = 307
Molecular formula = $C_{11}H_{22}N_3O_5P$

The infrared absorption spectrum of A53868 factor A (free acid) in KBr pellet is shown in the

accompanying drawing. Significant absorption maxima occur at the following frequencies ($cm^{-1}$):

3368, 3312 and 3300 (broad, strong), 3063 (weak), 2966 (medium to weak), 2833 (very weak), 2663 (weak), 1671 (strong), 1625 (shoulder), 1536 (strong), 1469 (weak), 1442 (weak), 1386 (medium to weak), 1340 (very weak), 1277 (very weak), 1240 (shoulder), 1207 (strong), 1157 (very weak), 1068 (medium), 1048 (strong), 918 (medium to weak), 797 (medium), 778 (medium), and 625 (weak).

Amino-acid analyses on samples of A53868 factor A hydrolyzed with 6N HCl gave the following results:

| Amino Acid | μMoles/mg Found | Theoretical | Average % Purity |
|---|---|---|---|
| glycine | 2.8 | 3.2 | 87% |
| leucine | 2.86 | 3.2 | 87% |

Electrometric titration of A53868 factor A in 66% aqueous dimethylformamide indicated the presence of a titratable group with a $pK_a$ value of 8.2 (the glycyl amino group).

A53868 factor A is soluble in water and dimethyl sulfoxide and is insoluble in most organic solvents.

Nuclear magnetic resonance spectrometry of A53868 factor A, using a 360 MHz instrument with the sample dissolved in DMSO-$d_6$, indicated that A53868 factor A has the structure shown in formula <u>1</u> above.

The formulae <u>1</u> and <u>2</u> compounds are capable of forming salts which are also part of this invention.

It will be appreciated that the formulae 1 and 2 compounds have both an acid function which can form salts and an amino group which can form acid-addition salts. Such salts are useful, for example, for separating and purifying the antibiotics. In addition, pharmaceutically acceptable salts are especially useful.

Pharmaceutically-acceptable alkali-metal, alkaline-earth-metal and amine salts and acid-addition salts are particularly useful. Representative and suitable alkali-metal and alkaline-earth metal salts include the sodium, potassium, lithium, cesium, rubidium, barium, calcium and magnesium salts. Suitable amine salts include the ammonium and the primary, secondary, and tertiary $C_1$-$C_4$-alkylammonium and hydroxy-$C_2$-$C_4$-alkylammonium salts. Illustrative amine salts include those formed by reaction of a formula 2 compound with ammonium hydroxide, methylamine, *sec*-butylamine, isopropylamine, diethylamine, cyclohexylamine, di-isopropylamine, cyclohexylamine, ethanolamine, triethylamine, 3-amino-1-propanol, and the like.

The alkali-metal and alkaline-earth-metal cationic salts are prepared according to procedures commonly used for the preparation of cationic salts. For example, an appropriate formula 2 compound is dissolved in a suitable solvent such as water; a solution containing the stoichiometric quantity of the desired inorganic base is added to this solution, using care to prevent the pH of the solution from becoming too basic. The salt thus formed can be isolated by routine methods, such as filtration or evaporation of

the solvent. Alternatively, the formula 2 compound in solution can be passed over an appropriate ion-exchange resin.

The salts formed with organic amines can be prepared in a similar manner. For example, the gaseous or liquid amine can be added to a solution of an appropriate formula 2 compound in a suitable solvent such as water; the solvent and excess amine can be removed by evaporation.

Representative and suitable acid-addition salts include those salts formed by standard reaction with both organic and inorganic acids such as, for example, hydrochloric, sulfuric, phosphoric, acetic, succinic, citric, lactic, maleic, fumaric, palmitic, cholic, pamoic, mucic, D-glutamic, *d*-camphoric, glutaric, glycolic, phthalic, tartaric, lauric, stearic, salicylic, methanesulfonic, benzenesulfonic, sorbic, picric, benzoic, cinnamic, and like acids.

The novel antibiotic of formula 1 is produced by culturing an A53868-factor A-producing strain of *Streptomyces luridus* under submerged aerobic conditions in a suitable culture medium until substantial antibiotic activity is produced. The antibiotic is recovered by the use of various isolation and purification procedures recognized in the fermentation art.

The new organism useful for the preparation of A53868 factor A was isolated from a soil sample collected from Granik Rapids at the bottom of the Grand Canyon in Arizona. This organism, called culture A53868, is classified as a strain of *Streptomyces*

*luridus*. This classification is based on a comparison with published descriptions of this species [R. E. Buchanan and N. E. Gibbons, "Bergey's Manual of Determinative Bacteriology," 8th Ed, The Williams and Wilkins Company, Baltimore, MD, 1974; E. B. Shirling and D. Gottlieb, "Cooperative Description of Type Cultures of *Streptomyces*," *Intern. Journal of Systematic Bacteriol. 18* (2): 142 (1968); E. Kuster, "Simple Working Key for the Classification and Identification of Named Taxa Included in the International *Streptomyces* Project", *ibid* *22* (3): 134-148 (1972); H. Nonomura, "Key for Classification and Identification of 458 Species of the Streptomycetes Included in ISP," *J. Ferment. Technol.* *52* (2): 78-92 (1974); I. M. Szabo et al., "A Diagnostic Key for the Identification of "Species" of *Streptomyces* and *Streptoverticillum* Included in the International Streptomyces Project," *Acta Botanica Academiae Scientiarium Hungaricae* *21* (3-4), 387-418 (1975); and S. A. Waksman, "The Actinomycetes" Vol. II, The Williams and Wilkins Co., Baltimore, MD, 1961, p. 236].

This classification is based on methods recommended for the International *Streptomyces* Project (ISP) [E. B. Shirling and D. Gottlieb, "Methods of Characterization of *Streptomyces* Species," *Intern. Journal of Systematic Bacteriol. 16* (3), 313-340 (1966)] along with certain supplementary tests.

Carbon utilization was determined with ISP #9 basal medium to which filter-sterilized carbon sources were added to equal a final concentration of 1.0%. The

basal medium was sterilized by autoclaving. Plates were read after 14 days incubation at 30°C.

The cell-wall sugars were determined using a modification of the procedure of Lechevalier (M. P. Lechevalier, "Chemical Methods as Criteria for the Separation of Actinomycetes into Genera," Workshop sponsored by the Subcommittee on Actinomycetes of the American Society of Microbiology, Dr. Thomas G. Pridham, Convenor; held at the Institute of Microbiology, Rutgers University, The State University of New Jersey, New Brunswick, NJ, 1971). The isomer of diaminopimelic acid was determined using the method of Becker *et al.* [B. Becker, *et al.*, "Rapid Differentiation Between *Nocardia* and *Streptomyces* by Paper Chromatography of Whole Cell Hydrolysates," *Appl. Microbiol.* *11*, 421-423 (1964)].

Melanoid pigment production (chromogenicity) was determined using ISP #1 (tryptone-yeast extract broth), ISP #6 (peptone-yeast extract iron agar), ISP #7 (tyrosine agar), and modified ISP #7 (ISP #7 without tyrosine).

Starch hydrolysis was determined by testing for the presence of starch with iodine on ISP #4 (inorganic salts-starch agar) plates (D. J. Blazevic and G. M. Ederer, "Principles of Biochemical Tests in Diagnostic Microbiology," John Wiley and Sons, New York, NY, 1975, p. 99).

Temperature range and NaCl tolerance were done using ISP #2 agar medium. NaCl tolerance was measured by adding NaCl to the agar to equal the desired concentrations. These were incubated at 30°C.

ISCC-NBS Centroid Color Charts, Standard Sample No. 2106 (U.S. Department of Commerce, National Bureau of Standards, Washington, D.C., 1958) and the Color Harmony Manual (4th Ed, Color Standards Dept., Container Corp. of America, IL, 1958) were used to assign color names.

Cultural Characteristics

A53868 produces abundant aerial mycelia with a spore mass color in the red (R) color series. The nearest matching color tab for the red color series in the Tresner and Backus system [Color Harmony Manual and H. D. Tresner, and E. J. Backus, "System of Color Wheels for Streptomycete Taxonomy," Appl. Microbiol. 11, 335-338 (1956)] is 5ca light yellowish pink to 4ec grayish-yellowish pink. In the ISCC-NBS system, the nearest matching color chip is 31.p.y Pink, pale yellowish pink. This cultural characteristic is produced on oatmeal agar (ISP No. 3), Czapek's-solution agar and tomato paste-oatmeal agar (TPO). It is best seen when grown on inorganic salts-starch agar (ISP No. 4).

The reverse of the colony produces no distinctive pigments. The color of the reverse side is a moderate orange yellow when grown on ISP No. 4. This color varies in shade and intensity when grown on other media. No soluble pigments are produced.

When plated for variability, this culture presented a stable homogeneous colony type. An occasional variant with no aerial hyphae was observed. This cultural information is detailed in Table I.

Table I

Cultural Characteristics of A53868

| Medium | | Characteristics[a] |
|---|---|---|
| ISP No. 2 | G | Abundant |
| | R | 68.S.OY |
| | Am | Abundant: 3CA Pale OY (R) |
| | Sp | None |
| ISP No. 3 | G | Good |
| | R | 33.br Pink |
| | Am | Fair: 4ec Grayish yellowish pink (R) |
| | Sp | None |
| ISP No. 4 | G | Abundant |
| | R | 71.m.OY |
| | Am | Abundant: 5CA Light yellowish pink (R) |
| | Sp | None |
| ISP No. 5 | G | Abundant |
| | R | 86.l.Yellow |
| | Am | Abundant: a White (W) |
| | Sp | None |
| Czapek's Agar | G | Fair |
| | R | 73.p.OY (very pale) |
| | Am | Fair: 3CA p.OY to 4ec gy.yPK (R) |
| | Sp | None |
| TPO | G | Abundant |
| | R | 72.d.OY |
| | Am | Abundant: 3CA p.OY to 5cb gy.yPK (R) |
| | Sp | None |

[a] G = growth R = reverse Am = aerial mycelia
Sp = soluble pigment

Morphological Characteristics

Culture A53868 produces well-developed, non-fragmenting aerial mycelia which are monopodially branched. Sporophores are of moderate length and are arranged as hooks and open loops of wide diameter. Occasional primitive spirals are also observed. When present, they are short, tight and compact. Sporophore morphology is placed in the section Retinaculiaperti (RA) of Pridham, *et al.*, *supra*.

This morphology is best observed on ISP No. 4 and Czapek's solution agar. Mature spore chains generally contain about 10 spores per chain. The spore shape is spherical to oblong, but is mainly oblong. The spore size was determined with an optical light microscope, using a Vickers Image Splitting Measuring Eyepiece. The spore size ranges from 0.93 - 1.85 μM in length and 0.62 - 1.30 μM in width. The average size is 1.28 μM x 0.94 μM. The spore surface ornamentation is smooth.

Physiological Characteristics

Analysis of hydrolyzed whole cells demonstrated the presence of LL-DAP (diaminopimelic acid) with no *meso* isomer present. Sugar analysis of hydrolyzed whole cells demonstrated the presence of glucose, mannose and ribose. This represents a Type I cell wall and an NC, or no-characteristic, sugar pattern (see "Bergey's Manual", *supra*). This combination of major cell-wall constituents is indicative of the genus *Streptomyces*.

The carbon utilization pattern for A53868 is as follows: L-arabinose, D-glucose, cellobiose, D-fructose, D-galactose, i-inositol, lactose, D-maltose, D-ribose, salicin, sodium acetate, sodium citrate, sodium succinate, and D-xylose are all utilized for growth. D-arabinose, melibiose, D-mannitol, D-raffinose, L-rhamnose, and sucrose do not support growth.

Culture A53868 will liquefy gelatin and hydrolyze starch. It does not reduce nitrate. Skim milk is neither hydrolyzed nor peptonized by the culture.

Culture A53868 will tolerate up to 7 percent NaCl and will grow at temperatures between 10-37°C.

Melanoid pigments are produced by A53868 when grown in tryptone-yeast extract broth (ISP No. 1), and on slants of peptone-yeast extract-iron agar (ISP No. 6) and tyrosine agar (ISP No. 7).

Species Determination

The cultural, morphological and physiological characteristics of A53868 were compared with published descriptions of similar species. Fourteen *Streptomyces* species had close resemblance to culture A53868 and were, therefore, studied in detail. Two species were selected as being the most similar to A53868. These two cultures are:

*Streptomyces lavendofoliae* (E.B. Shirling, et al., *supra*, p. 339)

*Streptomyces luridus* (E.B. Shirling, et al., *supra*, p. 142)

These two cultures are reported in the literature as belonging in the red (R) color series with retinaculiaperti (RA) sporophore morphology, smooth (Sm) spore-surface ornamentation, producing melanoid pigments and having a carbon-utilization pattern and other characteristics quite similar to A53868. They are both recognized in the Approved List of Bacterial Names [V.B.D. Skerman et al., Intern. Journal of Systematic Bacteriol. 30 (1), 225-420 (1980)].

Further comparison of culture A53868 with these two cultures shows:

S. lavendofoliae: This culture has many characteristics in common with A53868; and, although the differences are few, they are more than those with S. luridus. The general morphology, pigmentation, lack of soluble pigments, production of melanoid pigment, spore-mass color, spore-surface ornamentation and carbon utilization are all similar to A53868. S. lavendofoliae differs from A53868 in having a longer spore chain, more spiral sporophores, lack of fructose utilization, and poorer growth on Czapek's solution agar.

S. luridus: This culture is similar culturally, morphologically and physiologically to A53868. Both cultures are in the Red (R) color series, lack distinctive pigments, have the same (RA) sporophore morphology with smooth spore surface, produce melanoid pigments, and have a similar carbon utilization pattern. The similarities and differences between A53868 and

*S. luridus* are summarized in Table II. Tables III and IV give more detailed comparisons between A53868 and *S. luridus*.

Table II

Comparison of Culture A53868 and *S. luridus*

| Similarities | Differences |
|---|---|
| Aerial spore mass color (R) | Gelatin liquefaction |
| Carbon-utilization pattern | NaCl tolerance |
| Cultural characteristics | Nitrate reduction |
| Distinctive pigments absent | Skim-milk reaction |
| Melanoid pigments produced | Utilization of fructose |
| Morphology (RA) | |
| Soluble pigments absent | |
| Spore-chain length (10-50) | |
| Spore shape | |
| Spore-surface ornamentation | |
| Starch hydrolysis | |

Table III

Utilization of Carbon Compounds
by Culture A53868 and *S. luridus*[a]

| Carbon Source | A53868 | *S. luridus* |
|---|---|---|
| No carbon | - | - |
| L-arabinose | + | + |
| D-fructose | + | ± |
| D-glucose | + | + |
| i-inositol | + | + |
| D-mannitol | - | - |
| raffinose | - | - |
| L-rhamnose | - | - |
| sucrose | - | - |
| D-xylose | + | + |
| D-arabinose | - | ND[b] |
| cellobiose | + | ND |
| D-galactose | + | ND |
| lactose | + | ND |
| D-maltose | + | ND |
| melibiose | - | ND |
| Na-acetate | + | ND |
| Na-citrate | ± | ND |
| Na-succinate | + | ND |
| ribose | + | ND |
| salicin | + | ND |

[a] - = no utilization
\+ = utilization
± = doubtful utilization

[b] ND = not done

Table IV

Comparison of A53868 and *S. luridus*

| Characteristic | A53868 | *S. luridus* |
|---|---|---|
| Aerial spore mass color | Red | Red |
| Carbon-utilization pattern | + | + |
| (D-fructose) | + | ± |
| Cell-wall type | ± | ND[a] |
| Gelatin liquefaction | + | - |
| Melanoid pigment | + | + |
| ISP No. 1 | + | + |
| ISP No. 6 | + | + |
| ISP No. 7 | + | + |
| ISP No. 7 mod. | - | ND |
| Morphology | RA | RA |
| NaCl tolerance - percent | 7 | <7 |
| Nitrate reduction | - | + |
| Reverse side color | OY | OY |
| Skim milk | - | + |
| Soluble pigments | - | - |
| Spore shape | Oblong | Oblong |
| Spore surface | Sm | Sm |
| Starch hydrolysis | + | + |
| Temperature range - °C | 10-37 | ND |

[a]ND = not done

These comparisons indicate that A53868 is very similar to *S. luridus*. Culture A53868 is, therefore, classified as a strain of *Streptomyces luridus*

(Krasilnikov, Korenyako, Meksina, Valedinskaya and Veselov) Waksman 1961. This classification was based on a comparison with published descriptions and not on direct laboratory comparisons.

The *Streptomyces luridus* culture useful for the production of A53868 factor A has been deposited and made a part of the stock culture collection of the Northern Regional Research Center, U.S. Department of Agriculture, Agricultural Research Service, Peoria, Illinois, 61604, from which it is available to the public under the number NRRL 15101, date of deposit July 14, 1982

As is the case with other organisms, the characteristics of the A53868-factor-A-producing culture, *Streptomyces luridus* NRRL 15101, are subject to variation. For example, variants, recombinants, and mutants of the NRRL 15101 strain may be obtained by treatment with various known mutagens such as ultraviolet rays, X-rays, high-frequency waves, radioactive rays and chemicals. All natural and induced variants, mutants and recombinants of *Streptomyces luridus* NRRL 15101 which produce A53868 factor A may be used.

The culture medium used to grow *Streptomyces luridus* NRRL 15101 can be any one of a number of media. For economy in production, optimal yield, and ease of product isolation, however, certain culture media are preferred.. Thus, for example, a preferred carbon source in large-scale fermentation is soluble starch, although glucose, starch, dextrin, glycerol, and the like can also be used. A preferred nitrogen source is pancreatic digest of casein, although enzyme-

hydrolyzed casein, enzymatic digest of soy meal, acid-hydrolyzed casein, and the like are also useful. Glycine and leucine enrichment of the medium may also be beneficial.

Nutrient inorganic salts which can be incorporated in the culture media are the soluble salts capable of yielding calcium potassium, ammonium, chloride, sulfate, nitrate, phosphate, and like ions.

Essential trace elements necessary for the growth and development of the organism should also be included in the culture medium. Such trace elements commonly occur as impurities in other constituents of the medium in amounts sufficient to meet the growth requirements of the organism.

It may be necessary to add small amounts (e.g., 0.2 ml/L) of an antifoam agent such as polypropylene glycol to large-scale fermentation media if foaming becomes a problem.

For production of substantial quantities of A53868 factor A, submerged aerobic fermentation in tanks is preferred. Small quantities of A53868 factor A may be obtained by shake-flask culture. Because of the time lag in antibiotic production commonly associated with inoculation of large tanks with the spore form of the organism, it is preferable to use a vegetative inoculum. The vegetative inoculum is prepared by inoculating a small volume of culture medium with the spore form or mycelial fragments of the organism to obtain a fresh, actively growing culture of the organism. The vegetative inoculum is then transferred to a larger tank.

The A53868-factor-A-producing organism can be grown at temperatures between about 10° and about 37°C. Optimum A53868 factor A production appears to occur at temperatures of about 28°-30°C.

As is customary in aerobic submerged culture processes, sterile air is dispersed through the culture medium. For efficient production of A53868 factor A, the dissolved oxygen level should be maintained above 30% of air saturation (at 30°C. and atmospheric pressure).

For tank fermentation, it is preferable to maintain the pH level of the fermentation medium in a range of from about 6.5-7.4. This can be done by the addition of appropriate amounts of, for example, sodium hydroxide or hydrochloric acid.

Production of A53868 factor A can be followed during the fermentation by testing samples of the broth or of extracts of the mycelial solids for antibiotic activity against organisms known to be sensitive to the antibiotic. One assay organism useful in testing this antibiotic is _Micrococcus luteus_. The bioassay is preferably performed by paper-disc assay on agar plates.

Following its production under submerged aerobic fermentation conditions, A53868 factor A can be recovered from the fermentation medium by methods recognized in the fermentation art. The antibiotic activity produced during fermentation of an A53868-factor-A-producing organism generally occurs in the broth. Maximum recovery of A53868 factor A is accomplished, therefore, by an initial filtration to remove the mycelial mass. The filtered broth can be purified by a variety of techniques to give the A53868 complex.

A preferred method involves adsorption on a carbon column, eluting to give the A53868 complex.

Further purification and separation of the A53868 complex to give the individual A53868 factor A includes additional adsorption and extraction procedures. Useful adsorptive materials for the purification of the A53868 complex and A53868 factor A include: 1) high porous polymer (Diaion HP-20); 2) Sephadex A25 and G-50; Bio-Gel P-2 and P-10; 3) Anion-exchange resins - a) strongly basic; polystyrene, BioRad AG 1 & 2, Bio-Rex, Dowex 1 and 2, Amberlite IRA 400, 401, 410; b) moderately basic; epoxypolyamine Bio-Rex 5, and Duolite A30B; c) weakly basic; polystyrene or phenolic polyamine Bio-Rad AG3, Duolite A-6, A-7, Amberlite IRA 68, IR-45, IR-4B; 4) silica gel; 5) florisil; 6) polymeric adsorbents (XAD-2 and 4); 7) reversed-phase resins, silica gel/$C_{18}$ and silica gel/$C_8$; 8) carbon; 9) DEAE cellulose, DEAE Sephadex; 10) polyamide; 11) alumina; and 12) microcellulose. Sources: Bio-Rad and Bio-Gel resins - Bio Rad Laboratories, Richmond, CA; Amberlite and XAD resins -Rohm and Haas Co, Philadelphia, PA; Duolite resins - Diamond Shamrock Chemical Co, Redwood City, CA; Sephadex resins - Pharmacia Fine Chemicals AB, Uppsala, Sweden; Dowex resins - Dow Chemical Co., Midland, MI; Diaion-Mitsubishi Chemical Industries Ltd., Tokyo, Japan; XAD resins, silica gel/$C_{18}$ and silica gel/$C_8$ - E. Merck, Darmstadt, Germany.

The formula 2a compounds are prepared from compound 1 by routine methods for peptide synthesis.

The methods involve the coupling of amino acids or peptide fragments by reaction of the carboxyl function of one with the amino function of another to produce an amide linkage. In order to achieve coupling effectively, it is desirable: 1) that all reactive functionalities not participating directly in the reaction be inactivated by the use of appropriate blocking groups and 2) that the carboxyl function which is to be coupled be appropriately activated to permit coupling to proceed. All of this involves a careful selection of both reaction sequence and reaction conditions as well as utilization of specific blocking groups so that the desired peptide product will be realized. Each of the amino acids which is used to produce the compounds of this invention and which has the particularly selected protecting groups and/or activating functionalities is prepared by using techniques recognized in the peptide art.

Selected combinations of blocking groups are used at each point of the synthesis of the compounds of formula 2a. These particular combinations have been found to function most smoothly. Other combinations would operate in the synthesis of the compounds of formula 2a, although, perhaps, with a lesser degree of success. Thus, for example, benzyloxycarbonyl (CBz), *t*-butyloxycarbonyl (BOC), *t*-amyloxycarbonyl (AOC), *p*-methoxybenzyloxycarbonyl (MBOC), adamantyloxycarbonyl (AdOC), and isobornyloxycarbonyl can be used as amino-blocking groups in the synthesis of the compounds of formula 2a. Benzyl (Bzl) is generally used as the hydroxy-protecting group for the tyrosyl residue even

though others, such as p-nitrobenzyl (PNB), p-methoxybenzyl (PMB), and the like, could be used.

The carboxyl blocking groups used in preparing the compounds of formula 2a can be any of the typical ester-forming groups, including, for example, methyl, ethyl, benzyl, p-nitrobenzyl, p-methoxybenzyl, 2,2,2-trichloroethyl, and the like.

Coupling of the suitably protected N-blocked amino acid or peptide fragment with a suitably protected carboxy-blocked amino acid or peptide fragment in preparation of the compounds of formula 2a consists of rendering the free carboxyl function of the amino acid or peptide fragment active to the coupling reaction. This can be accomplished using any of several well recognized techniques. One such activation technique involves conversion of the carboxyl function to a mixed anhydride. The free carboxyl function is activated by reaction with another acid, typically a derivative of carbonic acid, such as an acid chloride thereof. Examples of acid chlorides used to form mixed anhydrides are ethyl chloroformate, phenyl chloroformate, sec-butyl chloroformate, isobutyl chloroformate, pivaloyl chloride, and the like. Isobutyl chloroformate is preferred.

Another method of activating the carboxyl function for the purpose of carrying out the coupling reaction is by conversion to its active ester derivative. Such active esters include, for example, a 2,4,5-trichlorophenyl ester, a pentachlorophenyl ester, a p-nitrophenyl ester, and the like. Another coupling

method available for use is the well-recognized azide coupling method.

Cleavage of selected blocking groups is necessary at particular points in the synthesis sequence used in preparation of the compounds of formula 2a. A chemist of ordinary skill in the art of peptide synthesis can readily select from representative protecting groups those groups which are compatible in the sense that selective cleavage of the product can be accomplished, permitting removal of one or more but less than all of the protecting groups present on the amino acid or peptide fragment. These techniques are well recognized in the peptide art. A fuller discussion of the techniques which are available for selective cleavage is provided in the literature [see Schröder and Lübke, The Peptides, Volume I, Academic Press, New York, (1965), especially the Table provided at pages 72-75].

Cleavage of carboxyl protecting groups can be accomplished by alkaline saponification. Relatively strong alkaline conditions, typically using an alkali-metal hydroxide, such as sodium hydroxide, potassium hydroxide, lithium hydroxide, and the like, are generally used to deesterify the protected carboxyl group. The reaction conditions under which saponification is accomplished are well recognized in the art. The carboxyl-blocking groups can also be removed by catalytic hydrogenolysis including, for example, hydrogenolysis in the presence of a catalyst such as palladium on carbon. Furthermore, in those instances in

which the carboxyl blocking group is p-nitrobenzyl or 2,2,2-trichloroethyl, deblocking can be accomplished by reduction in the presence of zinc and hydrochloric acid.

The amino-blocking groups are cleaved by treating the protected amino acid or peptide with an acid such as formic acid, trifluoroacetic acid (TFA), p-toluenesulfonic acid (TSA), benzenesulfonic acid (BSA), naphthalenesulfonic acid, and the like, to form the respective acid addition salt product. Cleavage of the amino-blocking group can also be accomplished by treating the blocked amino acid or peptide with a mixture of HBr or HCl and acetic acid to produce the corresponding hydrobromide or hydrochloride acid addition salt. The particular method or reagent which is used will depend upon the chemical or physical characteristics of the materials involved in the specific deblocking reaction.

The formula 2 compounds wherein n is zero and Z is hydrogen, the H-LAPP compounds, are especially useful intermediates. These compounds are prepared by removal of the glycyl group from compound 1. The glycyl group can be selectively removed by leucine amino peptidase (lap) or by selective acid hydrolysis to give the H-LAPP intermediate.

The general sequence for the preparation of the compounds of formula 2a can be depicted as follows. In the sequence the symbol "AA" represents an amino-acid residue and GLAPP represents A53868 factor A.

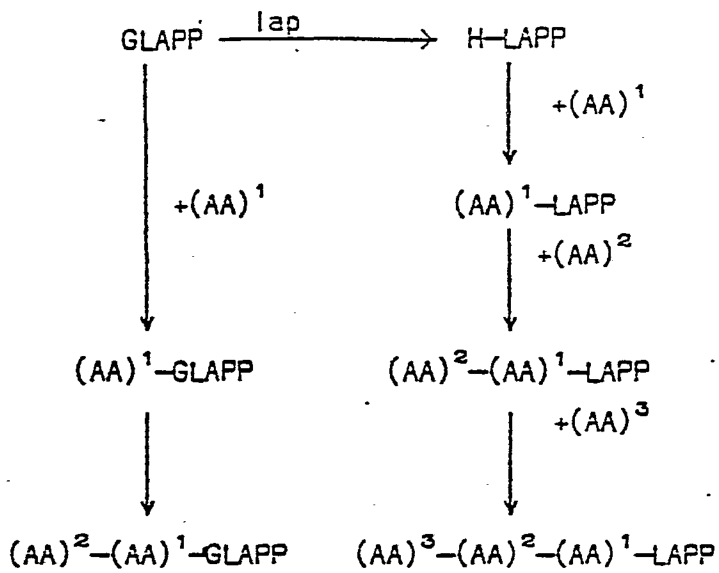


In preparing the compounds of formula <u>2a</u> by this sequence, it is preferable to use a compound which contains the group LAPP of the intended final product as the C-terminal reactant.

Examples of typical compounds of formula <u>2</u> include the following:

Ala-Gly-LAPP;
Tyr-Ala-Gly-LAPP;
Arg-Gly-LAPP;
Tyr-Arg-Gly-LAPP;
Nva-Gly-LAPP;
Tyr-Nva-Gly-LAPP;
Val-Gly-LAPP;
Tyr-Val-Gly-LAPP;
Nle-Gly-LAPP;
Tyr-Nle-Gly-LAPP;
Leu-Gly-LAPP;

Gly-Leu-Gly-LAPP;
Ile-Gly-LAPP;
Gly-Ile-Gly-LAPP;
Ala-Gly-LAPP;
Phe-Ala-Gly-LAPP;
Met-Ala-Gly-LAPP;
Leu-Ala-Gly-LAPP;
Ile-Ala-Gly-LAPP;
Lys-Gly-LAPP;
Pro-Gly-LAPP;
Thr-Gly-LAPP;
Trp-LAPP;
Val-Ala-Gly-LAPP;
Ser-Ala-Gly-LAPP;
Nle-Ala-Gly-LAPP;
Met-Gly-LAPP;
Tyr-Met-Gly-LAPP;
Phe-Met-Gly-LAPP;
Leu-Met-Gly-LAPP;
Ile-Met-Gly-LAPP;
Val-Met-Gly-LAPP;
Ser-Met-Gly-LAPP;
Ala-LAPP;
Tyr-LAPP;
Nle-LAPP;
Hse-LAPP;
Ala-Val-LAPP;
Cys-LAPP;
Cys-Gly-LAPP;
Hse-Gly-LAPP;
Val-Gly-LAPP;

Gly-Leu-LAPP;
Gly-Ala-LAPP;
Gly-Nle-LAPP;
Phe-Gly-LAPP;
Gly-Hse-LAPP;
Nva-Gly-LAPP;
Cys-Gly-LAPP;
Gly-Gly-LAPP;
Val-LAPP;
Ile-LAPP;
Arg-LAPP;
Asp-LAPP;
Gly-Asx-LAPP;
Gln-LAPP;
Glu-LAPP;
Glx-Gly-LAPP;
Leu-LAPP;
Nva-LAPP;
Phe-LAPP;
Ser-LAPP;

and the like.

This invention further relates to methods of regulating the immune system with, and compositions comprising, compounds of formulae 1, 2 and 3. The compounds of formula 3 are known in the art [see, for example, Hendlin et al., Science 166, 122 (1969)], but their immunomodulating activity was heretofore unrecognized. The compound of formula 3 wherein $R^2$ and $R^3$ are hydrogen is the antibiotic fosfomycin.

The compounds of formulae 1, 2 and 3 exhibit immunomodulating activity as demonstrated by *in vitro* and *in vivo* assays which test the ability of compounds to activate macrophages and by *in vivo* tests in animals with experimental intracellular infections. The compounds of formula 1 and 2 are a preferred group for this method. The related phosphonic acid antibiotics alaphosphin and fosmidomycin did not exhibit immunomodulatory activity in the *in vitro* assay.

The activity of the compounds of formulae 1, 2 and 3 on macrophage activation has been determined according to recognized procedures.

In one procedure, the ability of the compounds to activate murine macrophages *in vitro* was determined. Peritoneal macrophages were exposed to a compound of formula 1, 2, or 3 simultaneously with the addition of target cells. Tumor cytotoxicity of these treated macrophages was determined 48 hours later by trypan blue enumeration. The percentage of growth inhibition of P815 cells due to composition-mediated macrophage activation was calculated by comparison to that of P815 cells grown in the presence of macrophages exposed to buffer. The results of tests using this procedure are provided in Tables I and II:

Table I

Induction of Macrophage-Mediated Tumor Cytotoxicity by Compound 1

| Compound 1 Concentration (μg/ml) | Percent Macrophage Cytotoxicity Experiment 1 | Experiment 2 |
|---|---|---|
| 100 | 53 | NT[a] |
| 50 | 35 | 29 |
| 25 | 43 | 32 |
| 12.5 | 27 | 39 |
| 6.25 | 0 | 0 |
| 3.12 | 11 | 14 |
| 1.56 | NT | 0 |
| 0 | 0 | 0 |

[a]NT = not tested

Table II

Induction of Macrophage-Medicated Tumor Cytotoxicity by Fosfomycin

| Fosfomycin Concentration (μg/ml) | Percent Macrophage Cytotoxicity Experiment 1 | Experiment 2 |
|---|---|---|
| 100 | 47 | 30 |
| 10 | 49 | 11 |

In another procedure, peritoneal macrophages were harvested after 3, 5, or 10 days from composition-treated mice by peritoneal lavage and purified by adherence on plastic. Approximately $4 \times 10^5$ macrophages in 16-mm wells were overlaid with $4 \times 10^4$ P815 cells contained in 2 ml of Roswell Park Memorial Institute 1640 Medium supplemented with 20 percent fetal calf serum. All cultures were maintained in a humidified, 5-percent $CO_2$-in-air incubator at 37°C., and cytotoxicity was assessed at 48 hours on the basis of viable cell counts in a hemocytometer. Triplicate cultures were maintained for each group; the mean cell count and standard error (S.E.) were calculated. Under these conditions, peritoneal macrophages from normal BALB/c mice treated with tris-buffered saline did not affect the growth of P815 target cells, as measured both by viable cell number and by DNA synthesis of the leukemia cells. The ratio of macrophages to target cells was approximately 10:1 at the beginning of each experiment. The percentage of growth inhibition of P815 cells due to composition-mediated macrophage activation was calculated by comparison to that of P815 cells grown in the presence of macrophages from buffer-treated animals. The results of such a test are provided in Table III:

Table III

*In Vivo* Macrophage Activation Using Compound 1

| Compound 1 Dose (mg/kg) | Percent Macrophage Cytotoxicity at Day 0 | 3 | 5 | 10 |
|---|---|---|---|---|
| 100 | 0 | 43 | 25 | 0 |
| 10 | 0 | 8 | 20 | 0 |

In addition, the compounds of formulae 1, 2 and 3 have been found to be effective immunomodulators in tests in which both normal and immunosuppressed animals were infected with with intracellular infections in which the major cellular immunity is activated macrophages. Intracellular infections can be caused by bacteria, fungi, rickettsia, protozoa, and viruses including the following:

Mycobacterium tuberculosis,
Brucella,
Salmonella typhi,
Listeria monocytogenes,
Histoplasma capsulatum,
Candida albicans,
Chlamydiae,
Legionella,
Toxoplasma gondii,
Besnoitia jellisoni,
Plasmodium berghei,
Leishmaniasis, and
Herpes simplex.

In one test pretreatment with compound 1 was highly effective in protecting mice against a lethal Listeria monocytogenes EGD infections. In the test $CD_1$ mice weighing 18-20 g were pretreated parenterally with the test compound 5, 3, and 1 days before intravenous infection in the lateral tail vein with 0.1 ml containing $5 \times 10^5$ viable cells of Listeria monocytogenes EGD. Test compound was solubilized in pyrogen-free water and

administered intraperitoneally in 0.25 ml of the solution. Ten mice each were used for each concentration tested. Ten control mice were treated intraperitoneally at 5, 3, and 1 days preinfection with 0.25 ml of pyrogen-free water. The mice were caged, watered, and fed daily. The number of surviving mice was counted daily for 14 days. All surviving animals at 14 days were given an arbitrary 15 day survival time. The average survival time was calculated for each group. Significant differences between the treated and control groups of mice were determined by the standard "T" test.

Compound 1 was negative in the Limulus Lysate Test for endotoxin and was devoid of intrinsic *in vitro* antibacterial activity against *L. monocytogenes* EDG in a standard agar-diffusion test.

The results of the *in vivo* test vs. *Listeria monocytogenes* are summarized in Table IV.

Table VI

The Protective Effect of Compound 1 Pretreatment Against *Listeria monocytogenes* Mouse Infections

Percent Survivors

| Days Post Infection | Untreated Controls | Compound 1 Pretreatment (mg/kg) | | | | |
|---|---|---|---|---|---|---|
| | | 6.25 | 12.5 | 25 | 50 | 100 |
| 1 | 100 | 100 | 100 | 100 | 100 | 100 |
| 2 | 100 | 100 | 100 | 100 | 100 | 100 |
| 3 | 30 | 100 | 100 | 90 | 80 | 80 |
| 4 | 20 | 60 | 80 | 70 | 70 | 50 |
| 5 | 20 | 50 | 80 | 40 | 40 | 50 |
| 6 | 20 | 40 | 70 | 40 | 30 | 50 |
| 7 | 0 | 30 | 70 | 40 | 30 | 50 |
| 8 | 0 | 30 | 70 | 40 | 30 | 50 |
| 9 | 0 | 30 | 70 | 40 | 30 | 50 |
| 10 | 0 | 30 | 70 | 40 | 30 | 50 |
| 11 | 0 | 30 | 70 | 40 | 30 | 50 |
| 12 | 0 | 30 | 70 | 40 | 30 | 50 |
| 13 | 0 | 30 | 70 | 40 | 30 | 50 |
| 14 | 0 | 30 | 70 | 40 | 30 | 50 |
| 15 | 0 | 30 | 70 | 40 | 30 | 50 |
| Average Survival Time (Days) | 4.6 | 7.9* | 11.9* | 8.6* | 7.6* | 9.3* |

*Significantly different from untreated controls by "T" Test. P≤ 0.05

In another test, preinfection treatments with compound 1 were significantly effective in protecting mice from a lethal Candida albicans A26 infection. In this test $CD_1$ mice weighing 18-20 g were pretreated intraperitoneally or subcutaneously with test compound at 5, 3, and 1 days before intravenous infection with $5 \times 10^6$ viable cells of Candida albicans A26. Test compound was solubilized in pyrogen-free water and administered in 0.25 ml of solution. Ten mice were used for each concentration tested. Ten infected mice were treated intraperitoneally or subcutaneously at 5, 3, and 1 days preinfection with 0.25 ml of pyrogen-free water as controls. The immunomodulatory effects of the test compound were studied in X-irradiated mice exposed to 400R 24 hours preinfection, or mice treated intraperitoneally with 50 mg/kg cyclophosphamide 5, 3, and 1 days preinfection. The mice were caged, watered, and fed daily. The number of surviving mice was counted daily. Normal mice surviving 18 days post-infection were given an arbitrary 19-day survival time. X-irradiated or cyclophosphamide-treated mice surviving 12 days post-infection were given an arbitrary 13 day survival time. The average survival time was calculated for each group. Significant differences between treated and untreated groups of mice were determined by "T" test. Compound 1 was devoid of intrinsic in vitro antifungal activity against C. albicans A26 in a standard agar-diffusion test.

The results of in vivo tests vs. Candida albicans are summarized in Tables V-VII.

Table V

The Protective Effect of Compound 1 Pretreatment Against *Candida albicans* Infection in Normal Mice

| Compound 1 Dose (mg/kg) | Average Survival Time (Days) | Percent Increase In Average Survival Time Above Untreated Controls |
|---|---|---|
| 50 | 7.1 | 31.5* |
| 12.5 | 9.1 | 68.0* |
| Untreated Controls | 5.4 | -- |

*Significantly different from untreated controls by "T" Test. $P \leq 0.05$

Table VI

The Protective Effect of Compound 1 Pretreatment Against *Candida albicans* Infection in Cyclophosphamide-Pretreated Mice

| Compound 1 Dose (mg/kg) | Average Survival Time (Days) | Percent Increase In Average Survival Time Above Untreated Controls |
|---|---|---|
| 50 | 6.1 | 38.6* |
| 25 | 6.8 | 54.5* |
| Untreated Controls | 4.4 | -- |

*Significantly different from untreated controls by "T" Test. $P \leq 0.05$

Table VII

The Protective Effect of Compound 1 Pretreatment Against *Candida albicans* Infection in X-Irradiated Mice

| Compound 1 Dose (mg/kg) | Average Survival Time (Days) | Percent Increase In Average Survival Time Above Untreated Controls |
|---|---|---|
| 25 | 5.3 | 43.2* |
| 12.5 | 5.1 | 37.8* |
| Untreated Controls | 3.7 | -- |

*Significantly different from untreated controls by "T" Test. $P \leq 0.05$

Because they have the capability for correcting immune deficiencies in subjects in need of such correction, the compounds of formulae 1, 2 and 3 are therapeutically useful in the treatment of humans and animals. As a result, the compounds of formulae 1, 2 and 3 are considered to have multiple therapeutic uses. The compounds are considered useful in assisting the collective immunity of the body by increasing or assisting in therapeutic stimulation of cellular immunity. They would thereby become useful *in vivo* in the treatment of diseases involving chronic infection, such as fungal or mycoplasmal infections, tuberculosis, leprosy, acute and chronic viral infections, and the like. In one application, the immunomodulatory compounds of formulae 1, 2 and 3 may be administered in combination with one or more antimicrobial agents or antibiotics. Such combinations may be used prophylactically or therapeutically for control and/or treatment of microbial infections.

Further, the compounds are considered to be useful in any area in which cellular immunity is an issue and particularly where there are deficiencies in immunity such as in the Di-George Syndrome (congenital absence of thymus). Thus, they may be of therapeutic use in certain autoimmune disease in which damaging antibodies are present, for example, systemic lupus erythematosus, rheumatoid arthritis, and the like. Further, the compounds are useful in treatment of conditions in which the immune response is subnormal, such as neoplasia or organ transplantation. Administration of an effective immunoregulatory amount of the subject compounds will assist in the treatment of such conditions (especially neoplasia) either alone or in adjunct with other forms of treatment such as surgical removal.

One aspect of the present invention is, therefore, a method for regulating the immune system of a subject, human or animal, in need of such immune regulation which comprises administration to said subject an effective immunoregulatory amount of one of the subject compounds, preferably together with a pharmaceutical carrier. As used therein, the terms "regulate" or "modulate" mean that the compounds cause the immune system to support a normal, balanced state. In carrying out the methods of this invention, an effective amount of a compound of formula 1, 2 or 3 is administered parenterally to an infected or susceptible warm-blooded animal.

The dose which is effective to regulate the immune system will vary with the compound administered, the severity of the infection to which the immune system must respond, and the age, weight, and condition of the animal. The total dose required for protection parenterally will generally, however, be in the range of from about 5 to about 100 mg/kg and preferably will be in the range of from about 10 to about 50 mg/kg.

In another aspect, this invention relates to compositions useful for regulating the immune system in order to control intracellular infections. These compositions comprise a compound of formula 1 or 2 together with a suitable vehicle. Compositions may be formulated for parenteral administration by methods recognized in the pharmaceutical art.

Effective injectable compositions containing these compounds may be in either suspension or solution form. In the preparation of suitable formulations it will be recognized that, in general, the water solubility of the acid addition salts is greater than that of the free bases. Similarly, the bases are more soluble in dilute acids or in acidic solutions than in neutral or basic solutions.

In the solution form the compound is dissolved in a physiologically acceptable vehicle. Such vehicles comprise a suitable solvent, preservatives such as benzyl alcohol, if needed, and buffers. Useful solvents include, for example, water and aqueous alcohols, glycols, and carbonate esters such as diethyl carbonate. Such aqueous solutions contain, in general, no more than 50% of the organic solvent by volume.

Injectable suspension compositions require a liquid suspending medium, with or without adjuvants, as a vehicle. The suspending medium can be, for example, aqueous polyvinylpyrrolidone, inert oils such as vegetable oils or highly refined mineral oils, or aqueous carboxymethylcellulose.

Suitable physiologically acceptable adjuvants are necessary to keep the compound suspended in suspension compositions. The adjuvants may be chosen from among thickeners such as carboxymethylcellulose, polyvinylpyrrolidone, gelatin, and the alginates. Many surfactants are also useful as suspending agents. Lecithin, alkylphenol polyethylene oxide adducts, naphthalenesulfonates, alkylbenzenesulfonates, and the polyoxyethylene sorbitan esters are useful suspending agents.

Many substances which affect the hydrophilicity, density, and surface tension of the liquid suspending medium can assist in making injectable suspensions in individual cases. For example, silicone antifoams, sorbitol, and sugars can be useful suspending agents.

In order to illustrate more fully the preparation of the compounds of formulae 1 and 2 the following examples are provided.

## Example 1

### Preparation of Compound 1

### A. Shake-flask Fermentation of A53868

A lyophilized pellet of *Streptomyces luridus* NRRL 15101 is dissolved in 1-2 ml of sterilized water. This solution is used to inoculate an agar slant having the following composition:

| Ingredient | Amount (%) |
|---|---|
| Beef Extract | 0.05 |
| Glucose | 1.25 |
| Corn Starch | 0.5 |
| Potato Dextrin | 0.5 |
| Yeast Extract | 0.05 |
| Enzymatic Hydrolysate of casein[a] | 0.03 |
| Soluble Meat Peptone[b] | 0.5 |
| Blackstrap Molasses | 0.25 |
| $MgSO_4 \cdot 7H_2O$ | 0.025 |
| Czapek's Mineral Stock[c] | 0.2 |
| Deionized water | q.s. 1 liter |

Presterile pH adjusted to 7.5 with 5N NaOH; post-sterilization pH 6.8.

[a]NZ Amine A, Humko Sheffield Chemical, Lyndhurst, NJ.

[b]O.M. Peptone, Amber Laboratories, Juneau, WI 53039.

[c]Czapek's Mineral Stock has the following composition:

| | | |
|---|---|---|
| KCl | 10% | |
| $MgSO_4 \cdot 7H_2O$ | 10% | |
| $FeSO_4 \cdot 7H_2O$ | 2% | (dissolved in 2 ml of conc. HCl) |
| Deionized water | q.s. to 1 liter | |

The inoculated slant is incubated at 30°C. for about seven to fourteen days. The mature slant culture is covered with sterile distilled water (10 ml) and scraped with a sterile pipette to loosen the spores. A portion (0.5 ml) of the resulting suspension of spores is used to inoculate 50 ml of a vegetative medium having the following composition:

| Ingredient | Amount (%) |
|---|---|
| Glucose | 1.5 |
| Potato Dextrin | 2.0 |
| Soybean Grits | 1.5 |
| Yeast Extract | 0.1 |
| Corn-Steep Liquor | 1.0 |
| $CaCO_3$ | 0.2 |
| Cold Tap Water | q.s. to 1 liter |

Presterilization pH of about 5.6 adjusted to 6.5 with 5N NaOH; poststerilization pH 6.5-6.7.

The inoculated vegetative medium is incubated in a 250-ml Erlenmeyer flask at 30°C. for about 48 hours on a rotary shaker orbiting in a two-inch (5.08 cm) circle at 250 rpm.

Vegetative cultures have been successfully initiated with agar-slant cultures, with cultures preserved in liquid nitrogen and with lyophilized pellets of the culture.

Incubated vegetative medium (0.8 to 2%, volume/volume) is used to inoculate 50 ml of a production medium having the following composition:

| Ingredient | Amount (%) |
|---|---|
| Soluble Starch | 3.0 |
| Pancreatic Digest of Casein | 1.0 |
| $MgSO_4 \cdot 7H_2O$ | 0.05 |
| $K_2HPO_4$ | 0.05 |
| Ammonium Molybdate | 0.01 |
| $CaCO_3$ | 0.2 |
| Czapek's Mineral Stock[a] | 0.2 (2 ml/liter) |
| Deionized Water | q.s. to 1 liter |

[a]See Agar-slant medium, footnote (c)

The inoculated production medium was incubated in a 250-ml Erlenmeyer flask at 30°C. for 3-5 days on a two-inch rotary shaker at 250 rpm.

B. Tank Fermentation of A53868

In order to provide a larger volume of inoculum, 10 ml of incubated vegetative medium prepared as described above is used to inoculate 400 ml of a second-stage vegetative growth medium having the same composition as that of the vegetative medium. This second-stage medium is incubated in a two-liter flask for 48 hours at 30°C. on a two-inch rotary shaker at 250 rpm.

Incubated second-stage vegetative medium (2 L) thus prepared is used to inoculate 100 liters of

sterile production medium having the same composition given in Section A. The inoculated production medium is allowed to ferment in a 165-liter fermentation tank for about 4-6 days at a temperature of 30°C. The fermentation medium is stirred with conventional agitators and aerated with sterile air at a rate sufficient to maintain a dissolved oxygen level greater than 30% of air saturation at atmospheric pressure.

C. Separation of Compound 1

Fermentation broth (200 ml), obtained as described in Section B, was filtered, using a filter aid (2%, Hyflo, Johns-Manville Products Corp.). The mycelia were discarded, and the filtered broth was adsorbed onto a 16-liter Diaion HP-20 column (Mitsubishi Ind.). The column was eluted with 150 liters of acetonitrile:water (1:9), collecting 4-liter fractions. Elution of compound 1 was monitored by an HPLC assay. In this assay the compound was eluted from a 1/4''- x 30-cm Zorbax ODS (Dupont, 12 μ) column at 5.1 minutes under the following conditions:

| | | |
|---|---|---|
| flow rate | = | 2.0 ml/min |
| detection | = | UV at 254 nm |
| sensitivity | = | 0.16 Absorbance Units Full Scale |
| elution solvent | = | 8% $CH_3CN$ in 0.1% phosphate buffer, pH=3 |

The desired fractions were concentrated *in vacuo*, removing the acetonitrile to give an aqueous solution

containing 167 g (the concentration was determined by weighing dried aliquots) of crude product.

A portion of the aqueous crude product (50 g) was placed on a two-liter HP-20 column. The column was washed with water containing 10% NaCl (4 L) and then was eluted with acetonitrile:water (1:9), collecting 200-ml fractions. The fractions were analyzed by HPLC assay. The active fractions combined and concentrated under vacuum to remove acetonitrile and give additional aqueous concentrate containing about 15 g of crude product.

Portions of the aqueous concentrates (8-10 g) or the original HP-20 aqueous crude product were placed on a two-liter LP-1/$C_{18}$ preparative reverse-phase HPLC column, prepared as described by Abbott et al. in U.S. Patent 4,287,120, issued Sept. 1, 1981 (see Examples 6-7). After the column was washed with water (2 L), it was eluted with a solution of acetonitrile:water (5:95) plus 0.1% acetic acid (pH = 3) at a flow rate of 120 ml/min, collecting 80-ml fractions which were assayed by HPLC. Elution progress was monitored by UV at 254 nm. The desired fractions were combined and lyophilized to give more purified product (2-4 g).

This product was further purified as follows: Portions (1 g) were chromatographed over a one-liter Zorbax ODS (12 μ) preparative HPLC reverse-phase column, eluting with a solution of acetonitrile:water (5:95) plus 0.1% acetic acid (pH = 3) at a flow rate of 50 ml/min and collecting 25-ml fractions. Elution was monitored by UV at 254 nm, and fractions were analyzed by HPLC at 225 nm. The desired fractions were combined

and lyophilized to dryness to give 200-500 mg of purified material (85-95% pure).

The total yield of this material from 200 liters of fermentation broth was 5-6 g.

The purified material was subjected to a limulus ameobocyte lysate (LAL) test for endotoxin. If the test was positive, the material was filtered at 1 mg/ml in water through a Zeta Por filter (AMF CUNO, Meriden, CT) to remove the endotoxin. The filtrate was lyophilized to dryness to give a total of 3-5.6 g of purified endotoxin-free compound 1.

Endotoxin was also removed by hollow-fiber bundle dialysis into sterile pyrogen-free water. The material (about 500 mg) was dissolved in water (5 ml). This solution was cycled continuously through a 44-fiber Spectra Por HF hollow fiber bundle (Spectrum Medical Industries, Inc., New York, NY) with a molecular weight cut off of 5000 daltons. The hollow-fiber bundle was installed in a Fleaker hollow-fiber bundle dialysis apparatus (Corning Glass Works, Corning, NY) filled with 300 ml of sterile pyrogen-free water. The concentrate was cycled continuously through the hollow-fiber bundle at a rate of 1 ml/min, using an FMI pump (model RP, Fluid Metering Co., Oyster Bay, NY).

D. Characteristics of Compound 1

Form: colorless, amorphous

Empirical formula: $C_{11}H_{22}N_3O_5P$

Molecular weight: about 307

Elemental analysis:

| | Found | | Calculated for $C_{11}H_{21}N_3O_5PNa$ |
|---|---|---|---|
| | Sample I | Sample II | |
| Carbon | 41.32 | 38.74 | 40.12 |
| Hydrogen | 7.61 | 6.78 | 6.43 |
| Nitrogen | 11.53 | 12.42 | 12.76 |
| Oxygen | 24.38 | -- | 24.30 |
| Phosphorus | -- | 7.70 | 9.41 |
| Ash* | 13.58 | -- | -- |

*Ash was shown to be phosphate

Mass spectral analysis in the fast-atom bombardment mode:

| | m/Z | HRMS | Elemental Composition |
|---|---|---|---|
| M+H | 308 | 308.13818 | $C_{11}H_{23}N_3O_5P$ |
| | 143 | 143.11878 | $C_7H_{15}N_2O$ |
| | 138 | 138.03240 | $C_3H_9NO_3P$ |

Molecular weight = 307
Molecular formula = $C_{11}H_{22}N_3O_5P$

Infrared absorption spectrum (free acid) in KBr pellet shows significant absorption maxima at the following frequencies ($cm^{-1}$):

3368, 3312 and 3300 (broad, strong), 3063 (weak), 2966 (medium to weak), 2833 (very weak), 2663 (weak), 1671 (strong), 1625 (shoulder), 1536 (strong), 1469 (weak), 1442 (weak), 1386 (medium to weak), 1340 (very weak), 1277 (very weak), 1240 (shoulder), 1207

(strong), 1157 (very weak), 1068 (medium), 1048 (strong), 918 (medium to weak), 797 (medium), 778 (medium), and 625 (weak).

Amino-acid analyses (samples hydrolyzed with 6N HCl):

| Amino Acid | μMoles/mg Found | Theoretical | Average % Purity |
|---|---|---|---|
| glycine | 2.8 | 3.2 | 87% |
| leucine | 2.86 | 3.2 | 87% |

Electrometric titration (66% aqueous dimethylformamide): $pK_a$ of 8.2 (the glycyl amino group)

Solubility: soluble in water and dimethyl sulfoxide; insoluble in most organic solvents

Nuclear magnetic resonance (360 MHz instrument, sample dissolved in DMSO-$d_6$): indicates the structure shown in formula 1.

## Example 2

Compound 1 was prepared using the method of Preparation 1, but replacing the LP-1/$C_{18}$ purification column with the following procedure:

Crude product from the HP-20 step (180-200 g/200L fermentation) was stirred for 30 minutes in methanol (6-8 L.) and filtered. The undissolved solids were stirred in methanol again and filtered. The two methanol extracts were combined and concentrated (to 2 L.). This concentrate was added to 10 volumes of ethyl acetate, and the precipitate which formed was removed by filtration and dried to give 72-80 g of crude product.

Product obtained by this method (100 g) was dissolved in water (400-500 ml). This solution was adjusted to pH 10 by the addition of concentrated $NH_4OH$. The resulting solution was placed on a 2-L. ion-exchange column (AG1-X4 in the acetate form, 100-200 mesh, Dowex, packed and washed with water) at a rate of 20 ml/min. The column was then washed with water (4 L.) and eluted with 0.025% glacial acetic acid, collecting fractions having a volume of one liter. Fractions were monitored by analytical HPLC on a 1/4" x 15-cm Zorbax ODS (6μ) column, eluting with 7.5% acetonitrile in a 0.1% phosphate buffer (pH 3). The desired fractions were combined, concentrated and lyophilized to give 15.5 g of more purified product (80-90% purity).

This product was further purified by Zorbax ODS (12μ) preparative HPLC, as in Preparation 1, but eluting with an acetonitrile:water (5:95) plus 0.025% pentafluoropropionic acid solvent system.

## Example 3

### Preparation of H-LAPP

Compound 1 was reacted with leucine amino peptidase (Sigma Chemical Co., St. Louis, MO), pH 7.5, at room temperature for two hours. The reaction was monitored by HPLC. When compound 1 had been fully reacted, the reaction was worked up by HPLC on a Zorbax ODS column using 7.5% $CH_3CN$ in 0.1M phosphate buffer, pH 3.5, and water to give a 20% yield of N-(1-methylene-2-phosphonoethyl)leucinamide [H-LAPP].

M + H $\frac{m/Z}{251}$

Molecular weight = 250

Molecular formula = $C_9H_{19}N_2O_4P$

Example 4

Preparation of Ala-Gly-LAPP

Compound 1 is reacted with the mixed anhydride of alanine with isobutyl chlorformate according to standard procedures to give Ala-Gly-LAPP.

Example 5

Preparation of Trp-LAPP

H-LAPP is reacted with the N-hydroxysuccinimide ester of tryptophan by standard methods to give Trp-LAPP.

Example 6

Preparation of Ile-Gly-LAPP

Ala-Gly-LAPP is reacted with the mixed anhydride of isoleucine with isobutyl chlorformate using standard procedures to give Ile-Gly-LAPP.

Example 7

Preparation of $CH_3$-C(O)-NH-GLAPP

Compound 1 (GLAPP), 100 mg, was dissolved in 2.5 ml of pyridine. One ml of acetic anhydride was added to the solution and the mixture was stirred 3 hours at room temperature. The mixture was concentrated under nitrogen to one ml, two ml of water was added, and the solution frozen and lyopholized. Yield 115 mg of title product.

Mass spectral analysis in the fast-atom bombardment mode:

| | m/Z | |
|---|---|---|
| M + H | 350 | (indicating monoacylation) |
| M + Na | 372 | |
| M + K | 388 | |

Molecular weight = 349.

Example 8

Preparation of GLAPP-mono and dimethyl phosphono esters

Compound 1 (GLAPP), 100 mg, was dissolved in 3 ml of methanol. To the solution was added 0.5 ml of 1N hydrochloric acid with stirring, then 1 ml of distilled diazomethane in diethyl ether was slowly added with stirring. The mixture was stirred for 3 hours, then stood overnight. The methanol was removed under nitrogen, 10 ml of distilled water added, the solution frozen and lyopholized. Yield 75 mg of the compounds of formula 1 wherein $R^2$ and $R^3$ are methyl or one of $R^2$ and $R^3$ is methyl and the other is hydrogen.

Mass spectral analysis in the fast-atom bombardment mode:

| | m/Z |
|---|---|
| M + H | 336 |
| M + H | 322 |

Molecular weight monomethyl = 321

Molecular weight dimethyl = 335

CLAIMS

1. A compound of the formula

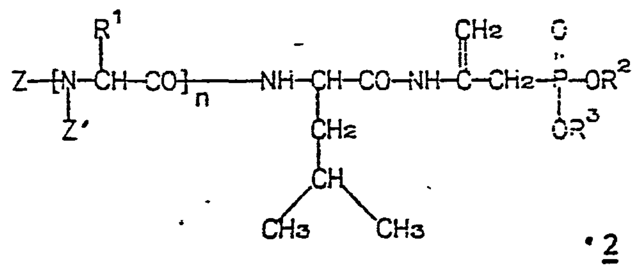

2

wherein

$R^1$ represents the characterizing group of an α-amino acid of the type normally found in proteins;

$R^2$ and $R^3$ are, independently, hydrogen or $C_1$-$C_3$-alkyl;

Z is hydrogen, $C_1$-$C_6$-alkyl, $C_1$-$C_6$-alkanoyl, phenyl-$C_1$-$C_3$-alkyl, phenyl-$(X)_m$-$C_1$-$C_3$-alkanoyl, or an amino-protecting group;

Z' is hydrogen or $C_1$-$C_6$-alkyl;

X is oxygen or sulfur;

m is 0 or 1; and

n is 0, 1, 2 or 3;

provided that when $R^2$ and $R^3$ are both alkyl, they must be identical; and the salts thereof.

2. A53868 factor A of claim 1 of the formula:

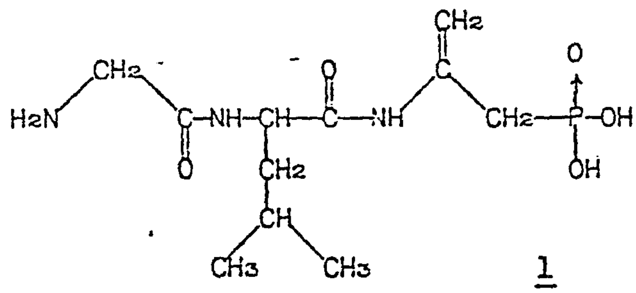

1

and the salts thereof.

3. A compound of claim 1 or 2 wherein the salt is pharmaceutically acceptable.

4. A compound of claim 3 wherein the phosphonate salt is the monosodium salt, the disodium salt, the monoammonium salt, the diammonium salt, or the monocyclohexylammonium salt.

5. The compound of claim 3 wherein the amine salt is the hydrochloride.

6. The compound of claim 1 wherein n is zero and Z, $R^2$ and $R^3$ are hydrogen.

7. A compound of claim 1 wherein n is one, Z, Z', $R^1$ and $R^2$ are hydrogen, and $R^3$ is a $C_1$-$C_3$ alkyl group.

8. The compound of claim 7 wherein $R^3$ is methyl.

9. The process for preparing A53868 factor A which comprises cultivating Streptomyces luridus NRRL 15101 or a mutant or recombinant thereof which produces A53868 factor A in a culture medium containing assimilable sources of carbon, nitrogen, and inorganic salts under submerged aerobic fermentation conditions until a

substantial amount of antibiotic activity is produced;

optionally isolating A53868 factor A; and

optionally reacting A53868 factor A by conventional methods of peptide synthesis to obtain the compounds of formula 2 as defined in claim 1.

10. The process of claim 9 which comprises cultivating Streptomyces luridus NRRL 15101.

11. The process of claim 9 or 10 which includes the step of isolating A53868 factor A.

12. An immunoregulatory pharmaceutical composition comprising, as active ingredient, an effective immunoregulatory amount of a compound of formula 2 as defined in claim 1, or a pharmaceutically acceptable salt thereof, together with one or more suitable pharmaceutical carriers.

13. A composition of claim 12 wherein the active ingredient is a compound of formula 1 as defined in claim 2.

14. A compound of formula 2, or a pharmaceutically-acceptable salt thereof, as claimed in claim 1, 3, 4, or 5, for use as an immunoregulatory agent.

15. A compound of formula 1, or a pharmaceutically-acceptable salt thereof, as claimed in claim 2, 3, 4 or 5, for use as an immunoregulatory agent.

16. A compound of formula 3:

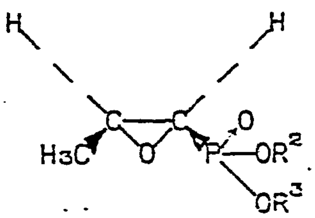

,

3

wherein $R^2$ and $R^3$, independently, are hydrogen or $C_1$-$C_3$-alkyl, or a pharmaceutically acceptable salt thereof, for use as an immunoregulatory agent.

17. The compound of claim 16 in which $R^2$ and $R^3$ are hydrogen.

18. The microorganism Streptomyces luridus NRRL 15101.

19. The microorganism Streptomyces luridus NRRL 15101 or a mutant or recombinant thereof which produces A53868 factor A.

CLAIMS

1. The process for preparing A53868 factor A which comprises cultivating <u>Streptomyces</u> <u>luridus</u> NRRL 15101 or a mutant or recombinant thereof which produces A53868 factor A in a culture medium containing assimilable sources of carbon, nitrogen, and inorganic salts under submerged aerobic fermentation conditions until a substantial amount of antibiotic activity is produced.

2. The process of claim 1 which comprises cultivating <u>Streptomyces</u> <u>luridus</u> NRRL 15101.

3. The process of claim 1 or 2 which includes the additional step of isolating A53868 factor A.

4. The process of claim 3 which includes the additional steps of reacting A53868 factor A with leucine amino peptidase or selective acid hydrolysis, followed by conventional solution phase peptide synthesis to provide the compounds of formula <u>2a</u>

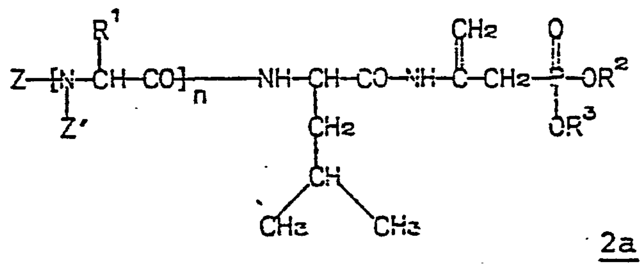

<u>2a</u>

wherein

$R^1$ represents the characterizing group of an α-amino acid of the type normally found in proteins;

$R^2$ and $R^3$ are, independently, hydrogen or $C_1$-$C_3$-alkyl;

Z is hydrogen, $C_1$-$C_6$-alkyl, $C_1$-$C_6$-alkanoyl, phenyl-$C_1$-$C_3$-alkyl, phenyl-$(X)_m$-$C_1$-$C_3$-alkanoyl, or an amino-protecting group;

Z' is hydrogen or $C_1$-$C_6$-alkyl;

X is oxygen or sulfur;

m is 0 or 1; and

n is 0, 1, 2 or 3;

provided that: 1) when $R^2$ and $R^3$ are both alkyl, they must be identical; and 2) when n is 1, one of $R^1$, $R^2$, $R^3$, Z or Z' must be other than hydrogen; and the salts thereof.

5. The process of claim 3 which includes the additional step of reacting A53868 factor A by conventional solution phase peptide synthesis to provide the compounds of formula 2a as defined in claim 4 wherein $R^1$ is hydrogen and Z or $Z^1$ is not hydrogen.

6. The process of claim 3, 4 or 5 which includes the additional step of derivatizing a compound of formula 2a wherein at least one of $R^2$, $R^3$, Z or $Z^1$ is not hydrogen.

7. A process of claim 1, 3, 4, 5 or 6 wherein the salt is pharmaceutically acceptable.

8. A process of claim 7 wherein the phosphonate salt is the monosodium salt, the disodium salt, the monoammonium salt, the diammonium salt, or the monocyclohexylammonium salt.

9. The process of claim 7 wherein the amine salt is the hydrochloride.

10. A compound of formula 1 as defined in claim 1, or a salt thereof, whenever prepared by a process according to any one of claims 1 to 3.

11. A compound of formula 2a as defined in claim 4, or a salt thereof, whenever prepared by a process according to any one of claims 4 to 6.

12. A biologically pure culture of *Streptomyces luridus* NRRL 15101.

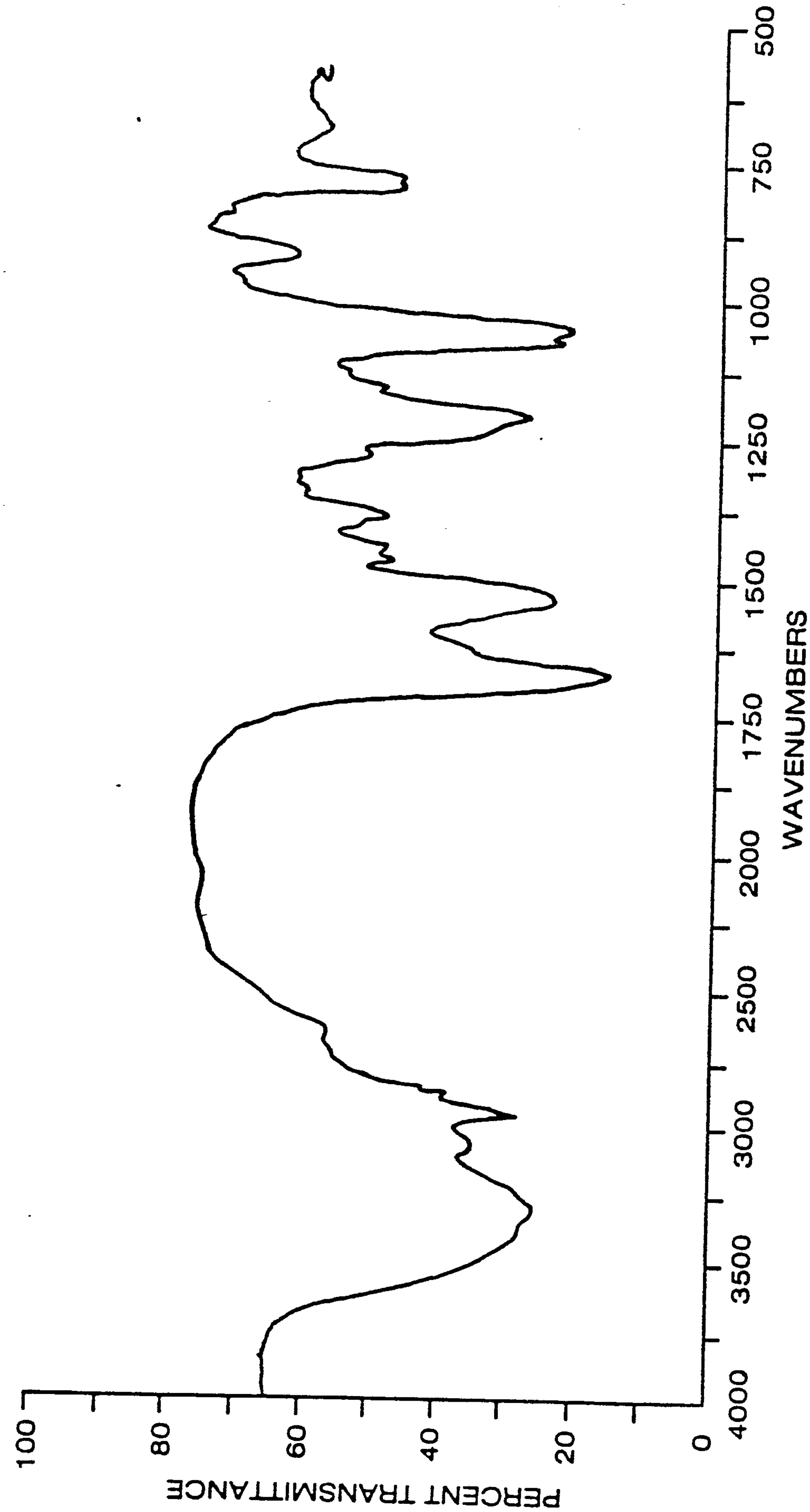
PERCENT TRANSMITTANCE
100
80
60
40
20
0
WAVENUMBERS
4000
3500
3000
2500
2000
1750
1500
1250
1000
750
500